# EUROPEAN PATENT SPECIFICATION

(11) **EP 1 147 762 B1**
(45) Date of publication and mention of the grant of the patent: **31.05.2006**
(21) Application number: 01109392.9
(22) Date of filing: 19.04.2001
(51) Int. Cl.: A61K 8/34, A61K 8/92, A61Q 5/12, A61K 8/37, A61K 8/58

(54) **Method of treating hair**
Verfahren zur Haarbehandlung
Procédé de traitement de la chevelure

(30) Priority: 19.04.2000 JP 2000118623
(43) Date of publication of application: 24.10.2001
(73) Proprietor: KAO CORPORATION, Chuo-ku, Tokyo (JP)
(72) Inventor: Ueyama, Kenichi, Sumida-ku, Tokyo (JP); Ito, Takashi, Sumida-ku, Tokyo (JP); Tsuchiya, Masaru, Sumida-ku, Tokyo (JP)
(74) Representative: HOFFMANN EITLE

(56) References cited:
- WO-A-00/38621
- WO-A-01/08654
- WO-A-02/060408
- DE-C- 4 215 501
- GB-A- 2 090 303
- US-A- 3 770 648
- US-A- 4 237 112
- US-A- 4 344 446
- US-A- 4 487 883
- US-A- 4 534 981
- US-A- 4 626 529
- US-A- 4 677 123
- US-A- 4 826 681

## Description

### FIELD OF THE INVENTION

The present invention relates to a hair treating method for imparting improved feel and stylability or manageability to hair.

### BACKGROUND OF THE INVENTION

Various hair treatments have been practiced for the purpose of leaving hair soft or elastic, conditioning or modifying hair, improving the feel, giving care to damaged hair, and the like.

These hair treatments have been done by applying various hair treatment preparations on shampooed wet hair, but conventional hair treating methods are not sufficiently effective in improving feel or stylability of hair.

Japanese Patent Laid-Open No. 11-79953 proposes a hair cosmetic preparation which is applied to styled hair to keep the hair in style with moderate set and leave the hair glossy without ruffling, which comprises a higher alcohol, an ester oil and a lower alcohol and contains substantially no water. Japanese Patent Laid-Open No. 8-24035 discloses a hair treatment method for temporarily deform hair, which comprises bringing hair into contact with gas containing steam of at least 75°C, the hair having been treated with an oil-containing composition and being under mechanical tension. US-A-4 344 446 describes a method for cleansing and conditioning the hair and scalp by applying an anhydrous stick-like scalp cleanser to the scalp, before treating the preferably wet hair with a cleansing and conditioning shampoo.

US-A-4 237 112 discloses a non-settling, anhydrous medicated hair and scalp conditioning composition consisting essentially of about 50 to about 94 wt.% petrolatum, 0.5 to 5 wt.% mineral wax, at least about 0.5 wt.% polyoxyethylene (8) dilaurate, 0.15 to 2.25 wt.% sulfur as a medicament and 1 to 30 wt.% of an organically modified hectorite clay gellant which consists of hectorite modified by propylene carbonate, stearalkonium chloride or dimethyl-di-(hydrogenated tallow) ammonium chloride and an organic liquid such as isopropyl myristate.

Neither of these publications has a mention of method for improving feel of stylability or hair easily by letting rinses, hair conditioners, and the like manifest their effects efficiently.

### SUMMARY OF THE INVENTION

An object of the present invention is to provide a method of treating hair for improving feel or stylability easily by letting rinses, hair conditioners, and the like manifest their effects efficiently, and to provide a preshampoo treatment composition.

The present invention accomplishes the above object by providing a method of treating hair which comprises applying a hair treatment composition comprising at least one oil agent selected from the group consisting of ester oils, triglyceride oils, hydrocarbon oils, vegetable oils, animal oils, cetanol, myristyl alcohol, stearyl alcohol, behenyl alcohol, 2-octyldodecanol and a mixture thereof, and a solvent and having a water content of 20 % by weight or lower to dry hair, warming the hair at a temperature of from 38 to 65 °C for a prescribed period of time with the hair being covered so as not to allow a component of the hair treatment composition to escape, and washing the hair treatment composition away.

### BRIEF DESCRIPTION OF THE DRAWINGS

The present invention will be more particularly described with reference to the accompanying drawing, in which:

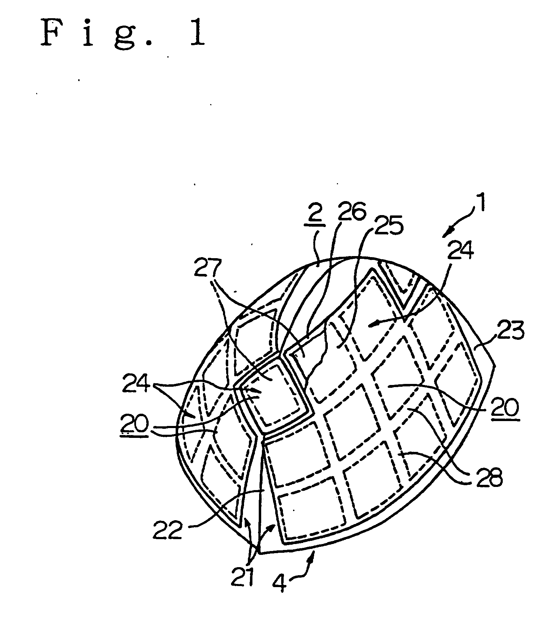

Fig. 1 is a hair warming cap which is preferably used in carrying out the present invention.

### DETAILED DESCRIPTION OF THE PREFERRED EMBODIMENTS

The hair treatment composition which can be used in the hair treating method according to the invention is described first.

The hair treating method is carried out by using a hair treatment composition comprising at least one oil agent and a solvent and having a water content of 20% by weight or lower.

Oil agents that have commonly been used in various hair treatment preparations or scalp treatment preparations can be used in the present invention. Examples of useful oil agents includes ester oils, such as isopropyl palmitate, isopropyl myristate, octyl palmitate, cetyl palmitate, propylene glycol dicaprate, neopentyl glycol dicaprate, neopentyl glycol di(2-ethylhexanoate), and isononyl isononanoate; triglycide oils, such as glycerol tri(2-ethylhexanoate) and glycerol tricaprate; cetanol, myristyl alcohol, stearyl alcohol, behenyl alcohol, and 2-octyldodecanol; hydrocarbon oils, such as liquid paraffin; and vegetable and animal oils (including essential oils). Preferred of them are ester oils such as isopropyl palmitate, isopropyl myristate, neopentyl glycol dicaprate, and isononyl isononanoate, and cetanol. These oil agents can be used either individually or as a combination of two or more thereof.

The solvent which can be used preferably includes components, which are liquid at ambient temperature and carry a hydroxyl group, such as lower alcohols having 1 to 6 carbon atoms (e.g. aromatic alcohols such as benzyl alcohol and benzyloxyethanol; polyhydric alcohols such as glycerol, propylene glycol and dipropylene glycol); alkylene carbonates such as propylene carbonate; and N-methylpyrrolidone. It is preferred for the composition to contain at least one of a lower alcohol having 1 to 6 carbon atoms (e.g., ethanol and isopropanol) and an aromatic alcohol (e.g., benzyloxyethanol and benzyl alcohol).

The hair treatment composition preferably comprises 0.5 to 50% by weight, particularly 1 to 25% by weight, of the oil agent and 3 to 99% by weight, particularly 5 to 50% by weight, of the solvent both based on the total composition. A total proportion of the oil agent and the solvent in the composition is preferably 5.5 to 100% by weight, particularly 10 to 70% by weight.

The hair treatment composition has a water content of not more than 20% by weight. The lower the water content, the preferred. A preferred water content is 15% by weight or lower, particularly 10% by weight or lower. The water content can be 0% by weight. If the water content exceeds 20% by weight, the composition fails to exhibit its full effect in modifying the surface of hairs, resulting in a failure to produce the effects of the present invention.

If desired, the hair treatment composition can contain other components in amounts that do not impair the effects of the present invention. For example, the composition can contain 0.01 to 50% by weight of surfactants, such as cationic, nonionic, anionic or amphoteric ones, as a solubilizer or an emulsifier; 0.01 to 10% by weight of cationic polymers (e.g., cationic cellulose derivatives), anionic polymers (e.g., polyacrylic acid), or nonionic polymers as a viscosity improver; and 0.01 to 20% by weight of polyhydric alcohols (e.g., glycerol) as a humectant. Additionally, various extracts, colorants, antiseptics, and antioxidants can be added to the composition in an amount of 0.01 to 5% by weight each.

The hair treating method according to the invention comprises applying the above-described hair treatment composition to dry hair.

If the hair treatment composition is applied to wet hair, for example, hair after shampooing, the effect of the composition in modifying the surface of hairs is not manifested to the full, resulting in a failure to obtain the effects of the present invention.

The term "dry hair" as used here is intended to exclude wet hair, such as undried hair after shampooing and hair wetted by water spraying. "Dry hair" may be quantitatively represented by a water content of not more than 30% by weight, preferably 20% by weight or less.

The hair treatment composition can be applied to hair in any way. For example, it is put once on a hand and then applied to hair, or it is applied to hair directly from a container, or it is sprayed over hair by use of a spray, etc.

In the invention, the hair wetted with the hair treatment composition is left to stand for 3 to 120 minutes, preferably 5 to 60 minutes, with warming and then shampooed. If the standing time is shorter than 3 minutes, the effect of the composition in modifying the surface of hairs is not manifested sufficiently, resulting in a failure to obtain the effects of the present invention. Even if the standing time is longer than 120 minutes, no further enhancement of effect results.

It is preferred that the hair having the hair treatment composition applied on be covered with, for example, a common shower cap or a sheet substantially impervious to moisture or water vapor, so that a component of the composition may not substantially escape, thereby to ensure the hair surface modifying effect of the composition.

After a lapse of a prescribed time period, the hair treatment composition is washed away with warm water or water in a usual manner. A shampoo can be used to wash off the hair treatment composition, and shampooing can be followed by conditioning with a rinse.

The hair treatment method makes rinses, hair conditioners, etc. applied after shampooing manifest their effects efficiently in imparting an improved feel (e.g., freedom from stickiness, smoothness, moist feel, etc.) or stylability to the hair.

It is considered that the hair treatment composition applied to dry hair efficiently renders the hair surface lipophilic to help the active ingredients of rinses, hair conditioners, etc., such as oil agents, be adsorbed onto the hair efficiently thereby bringing about the above-described effect.

In the hair treating method according to the present invention, hair to which the hair treatment composition has been applied is warmed at a prescribed temperature for a prescribed period of time and then washed.

By warming the hair having the hair treatment composition applied on, the effect of the composition on hair surface modification is enhanced, and its duration is prolonged.

Particularly for the purpose of prolonging the modifying effect, the hair warming time is preferably 3 minutes or longer, particularly 5 to 60 minutes, and the hair warming temperature is 38 to 65°C. The term "warming temperature" as used herein means the temperature of the hair while being warmed.

In this hair treatment method, hair is warmed while covered so that a vaporized component of the applied hair treatment composition may not substantially escape during warming. Unless hair is covered, the solvent tends to evaporate, and the expected effect is not developed efficiently particularly when the warming temperature is 45 to 65°C.

Warming hair while covered can be carried out by, for example, putting a conventional shower cap on the head or covering hair with a sheet substantially impervious to moisture or water vapor, and warming the hair with a known external heating tool (e.g., a hair drier) or a warming tool designed exclusively for hair which is a cap having a heating part. For ease and convenience for warming, the warming tool is used preferably.

Fig. 1 shows a warming cap for hair (hereinafter "hair warming cap") which can be used preferably for carrying out the hair treatment method. The hair warming cap 1 shown in Fig. 1 comprises a cap 2 having heating parts 20. The cap 2 is made of two sheets 21 of approximately the same shape and size. The heating parts 20 are provided by disposing heat transfer members 24 at prescribed positions on the sheets 21.

The heat transfer members 24 are each composed of an adhesive sheet 26 having a pressure-sensitive adhesive applied on the back side thereof, an air-permeable sheet 25, and a heat generating material 27 which is held between the sheets 25 and 26. The periphery of the heat generating material 27 is surrounded by a sealed area 28 formed by sealing the sheets 25 and 26. The heat transfer members 24 are each attached to an inner sheet 23 of the sheet 21 on their adhesive sheet 26.

The cap 2 is formed by joining the arc-shaped edges 22 of the two sheets 21 by, for example heat-sealing, to make a shape with an opening 4 in which a head is accommodated. The inner sheet 23 forms the inner side of the cap 2.

The heat generating material 27 includes a heat generating composition which mainly comprises iron powder and generates heat on contact with air, which is known as a component of a disposable body warmer, an electrical heating element which generates heat on electricity application, a heating element which generates heat on contact with water, and a heat retaining material which has a melting point and is capable of storing heat, such as polyethylene glycol.

The inner sheet 23 is made of a material having both water resistance and flexibility, for example, polyethylene, polypropylene or polyvinyl chloride. The air-permeable sheet 25 and the adhesive sheet 26 are the same as those used in common disposable body warmers. The adhesive sheet 26 can be omitted.

Where the above-described heat generating composition is used as a heat transfer material, the temperature and the duration of heat generation can be controlled by appropriately designing the composition and the air permeability of the air-permeable sheet 25.

The hair warming cap 1 is folded up into a compact form and packaged in an air-impermeable bag for the market in the same manner as with common disposable body warmers. On use, the package is opened to take out and unfold the hair warming cap 1. Simply fitting the hair warming cap 1 over the head achieves both covering and warming the hair.

The hair treating method according to the present invention brings about prolongation of the duration of the effect. That is, the number of times of shampooing and treatment with rinses or conditioners that can be repeated until the effect of the hair treating method (in modifying the hair surface) comes to an end is remarkably increased.

The preshampoo treatment composition used in the present invention efficiently makes the surface of hairs lipophilic to assist the oil agent component, etc. of rinses, hair conditioners, and the like that are used after shampooing in being adsorbed on the hairs. With respect to a preferred composition of the preshampoo treatment composition, the hair warming temperature and time, the manner of covering the hair, and the like, the corresponding description given applies.

The present invention will now be illustrated in greater detail with reference to Examples. The Examples are presented as being exemplary of the present invention and should not be construed as limiting. Unless otherwise noted, all the percents are by weight.

### EXAMPLES 1 TO 11

Hairs collected from Japanese females which had not experienced any chemical treatment such as a perm or bleach were bundled to prepare 20 cm long hair bundles each weighing 20 g for evaluation. The hair bundles were thoroughly shampooed, thoroughly rinsed, and completely dried to a water content of not more than 15%. Ten grams of the hair treatment composition shown in Tables 1 and 2 below was applied to each hair bundle, and the bundle was wrapped tightly in a wrap film, left to stand in a thermostat kept at room temperature (22°C) or 45°C for a prescribed period of time (see Tables), and rinsed with running water at 40°C for 30 seconds.

The treated hair bundles were shampooed with a commercially available shampoo (Lavinus Silky Feel Shampoo, available from Kao Corp.), conditioned with a commercially available conditioner (Lavinus Silky Feel Rinse, available from Kao Corp.), and thoroughly dried with a hair drier. A panel of 10 expert members organoleptically evaluated the thus treated hair bundles for freedom from stickiness, smoothness, moist feel, and stylability (or manageability) and gave them scores on a 1-to-5 scale in comparison with a standard sample which had been shampooed and conditioned in the same manner as described above but with no preshampoo treatment. Samples judged superior to the standard sample scored 5 (very good) or 4 (a little good). Samples judged equal to the standard sample scored 3. Samples judged inferior to the standard sample scored 2 (a little bad) or 1 (very bad). An average of the scores was worked out and graded according to the following standards.
A (Very good).... Average score of 4.5 or higher.
B (Good)............ Average score of 3.5 or higher and lower than 4.5.
C (Fair)..... Average score 2.5 or higher and lower than 3.5 (the test sample is regarded as being equivalent to the standard sample).
D (Poor)..... Average score lower than 2.5.

Further, the hair bundles after the above test were repeatedly subjected to a cycle of shampooing→conditioning→drying, and organoleptically evaluated in the same manner as described above after every cycle. Duration of the effect of the preshampoo hair treatment was evaluated by the number of the cycles repeated until the hair bundle was graded down from A or B to C for every item. For example, duration of "3" means that a sample was graded down to C after 3 cycles.The results obtained are shown in Tables 1 and 2.

**TABLE 1**

| | | Example | | | | | | |
|---|---|---|---|---|---|---|---|---|
| | | 1 (reference) | 2 | 3 | 4 | 5 | 6 | 7 (reference) |
| Hair Treatment Composition (wt%): | | | | | | | | |
| Oil agent | Isopropyl palmitate | 5 | 5 | 5 | 5 | 5 | 5 | 5 |
| | Ethanol | 95 | 95 | 95 | 10 | | 10 | 10 |
| Solvent | Benzyloxy-ethanol | | | | | 10 | 10 | 10 |
| | Dipropylene glycol | | | | 85 | 85 | 75 | 75 |

| Treating Conditions: | | | | | | | | |
|---|---|---|---|---|---|---|---|---|
| Temperature (°C) | | 22 | 45 | 45 | 45 | 45 | 45 | 22 |
| Time (min) | | 30 | 10 | 30 | 30 | 30 | 30 | 30 |

| Results of Evaluation: | | | | | | | | |
|---|---|---|---|---|---|---|---|---|
| Freedom from stickiness | | B | B | A | A | A | A | B |
| Smoothness | | B | B | A | A | A | A | B |
| Moist Feel | | B | B | B | B | B | B | B |
| Stylability | | B | B | A | A | A | A | B |
| Duration of effect | | 3 | 10 | 15 | 10 | 5 | 15 | 3 |

**TABLE 2**

| | | Example | | | |
|---|---|---|---|---|---|
| | | 8 | 9 | 10 | 11 |
| Hair Treatment Composition (wt%): | | | | | |
| Oil agent | Isopropyl Palmitate | 0 | 0 | 0 | 0 |
| | Neopentyl Glycol Dicaprate | 5 | 0 | 0 | 0 |
| | Cetanol | 0 | 5 | 0 | 0 |
| | Liquid Paraffin | 0 | 0 | 5 | 0 |
| | Octamethyl-siloxane | 0 | 0 | 0 | 5 |
| Solvent | Ethanol | 95 | 95 | 95 | 95 |
| | Benzyloxy-ethanol | 0 | 0 | 0 | 0 |
| | Dipropylene Glycol | 0 | 0 | 0 | 0 |

| Treating Conditions: | | | | | |
|---|---|---|---|---|---|
| Temperature (°C) | | 45 | 45 | 45 | 45 |
| Time (min) | | 30 | 30 | 30 | 30 |

| Results of Evaluation: | | | | | |
|---|---|---|---|---|---|
| Freedom from Stickiness | | A | B | B | B |
| Smoothness | | A | A | C | B |
| Moist Feel | | B | A | B | C |
| Stylability | | A | B | B | B |
| Duration of effect | | 20 | 15 | 15 | 15 |

### EXAMPLE 12

A panel of expert 10 members wearing their hair semi-long carried out the following organoleptic test on the hair treatment composition of Example 6. Of the ten members three were conscious of having damaged hair, four slightly damaged hair, and three little damaged hair. Thirty grams of the hair treatment composition was uniformly applied to the right half of their dry hair (water content: 15% or lower), with the left half left intact. The hair warming cap shown in Fig. 1 which had an iron powder-based heat generating material capable of generating heat on contact with air was put on the head and allowed to stand for 30 minutes with its opening edge sealed so that the solvent vapor might not escape. The formulation and amount of the heat generating material were adjusted so that the temperature of the hair on the right half of the head might be kept at 43 to 47°C.

After 30 minute warming, all the hair was thoroughly rinsed with running warm water (40°C) and treated with the same shampoo and then with the same conditioner as used in Examples 1 to 11, and completely dried. The panel evaluated their hair on the right half of their head in comparison with the hair on the left half as a standard according to the same scoring system as in the foregoing Examples. After the above organometallic evaluation, the panel repeated the cycle of shampooing→conditioning→drying 5 times and made the same evaluation. The results obtained were shown in Table 3. As can be seen from Table 3, the effect of the present invention has been demonstrated with significance in the test on actual living hair as well as on bundles of cut hairs. Prolongation of the duration of the effect was also confirmed.

**TABLE 3**

| | After Shampooing and Conditioning | After 5 Cycles After 5 Cycles |
|---|---|---|
| Freedom from stickiness | A | A |
| Smoothness | A | B |
| Moist feel | B | B |
| Stylability | A | A |

### COMPARATIVE EXAMPLES 1 TO 4

The same hair bundles as prepared in Examples 1 to 11 were treated and evaluated in the same manner as in Example 3, except for using the hair treatment compositions shown in Table 4. The results obtained are also shown in Table 4.

**TABLE 4**

| | | Comparative Example | | | |
|---|---|---|---|---|---|
| | | 1 | 2 | 3 | 4 |
| Hair Treatment Composition (wt%): | | | | | |
| Oil agent | Isopropyl palmitate | 5 | 5 | 100 | 0 |
| Solvent | Ethanol | 74 | 10 | 0 | 10 |
| | Benzyloxy-ethanol | 0 | 10 | 0 | 10 |
| | Dipropylene glycol | 0 | 54 | 0 | 80 |
| Purified Water | | 21 | 21 | 0 | 0 |

| Treating Conditions: | | | | | |
|---|---|---|---|---|---|
| Temperature (°C) | | 45 | 45 | 45 | 45 |
| Time (min) | | 30 | 30 | 30 | 30 |

| Results of Evaluation: | | | | | |
|---|---|---|---|---|---|
| Freedom from stickiness | | C | C | C | C |
| Smoothness | | C | C | C | C |
| Moist Feel | | C | C | C | C |
| Stylability | | B | B | C | C |
| Duration of effect | | 1 | 1 | 1 | 1 |

### EXAMPLE 13

A panel of expert 10 members wearing their hair semi-long (half of them were conscious of having damaged hair, and the rest were conscious of having slightly damaged hair) used a commercially available shampoo (Lavinus Silky Feel Shampoo, available from Kao Corp.) and a commercially available conditioner (Lavinus Silky Feel Rinse, available from Kao Corp.) for a week.

Fifty grams of a preshampoo treatment composition having the following formulation was uniformly applied to their dry hair (water content: not more than 15%).

| Formulation: | |
|---|---|
| Isopropyl palmitate | 1.5% |
| Neopentyl glycol dicaprate | 3.5% |
| Ethanol | 10% |
| Benzyloxyethanol | 10% |
| Dipropylene glycol | 75% |

A commercial shower cap was put on the head, and the hair was warmed at 43 to 47°C for about 30 minutes by use of a hair heating appliance (Rollerball, supplied by Takara Belmont). The hair was thoroughly rinsed with running warm water (40°C), treated with the same shampoo and the same conditioner as used above, and dried completely.

The preshampoo-treated and dried hair was scored by the panel in comparison with their hair before the treatment with the preshampoo treatment composition and graded based on the average score in the same manner as in Examples 1 to 11. The results, which are shown in Table 5 below, demonstrated the effects of the preshampoo treatment composition.

After the above organometallic evaluation, the panel repeated the cycle of shampooing→conditioning→drying 5 times and made the same evaluation. The results obtained are shown in Table 5, which provide confirmation of the effect on duration prolongation.

**TABLE 5**

| | After Shampooing and Conditioning | After 5 Cycles |
|---|---|---|
| Freedom from stickiness | A | A |
| Smoothness | A | A |
| Moist feel | A | B |
| Stylability | A | A |

The hair treating method of the present invention makes hair treatment preparations such as rinses, hair conditioners, and the like manifest their effects efficiently to give hair an improved feel (e.g., freedom from stickiness, smoothness, and moist feel) and stylability. In particular, the method of the invention sustains the effect of the hair treatment.

The invention having been thus described, it will be obvious that the same may be varied in many ways. Such variations are not to be regarded as a departure from the scope of the invention, and all such modifications as would be obvious to one skilled in the art are intended to be included within the scope of the following claims.

## Claims

1. A non-therapeutic method of treating hair which comprises applying a hair treatment composition comprising at least one oil agent selected from the group consisting of ester oils, triglyceride oils, hydrocarbon oils, vegetable oils, animal oils, cetanol, myristyl alcohol, stearyl alcohol, behenyl alcohol, 2-octyldodecanol and a mixture thereof, and a solvent and having a water content of 20 % by weight or lower to dry hair, warming the hair at a temperature of from 38 to 65 °C for a prescribed period of time with the hair being covered so as not to allow a component of the hair treatment composition to escape, and washing the hair treatment composition away.

2. The method of treating hair according to claim 1, wherein said warming the hair is carried out for 3 minutes or longer.

3. The method of treating hair according to claim 1 or 2, wherein said warming the hair is carried out with a hair warming cap having heating parts.

## Patentansprüche

1. Nicht-therapeutisches Verfahren zur Behandlung von Haar, umfassend das Auftragen einer Haarbehandlungszusammensetzung, die mindestens ein öliges Agens, das aus der Gruppe ausgewählt ist, die aus Esterölen, Triglyceridölen, Kohlenwasserstoffölen, pflanzlichen Ölen, tierischen Ölen, Cetanol, Myristylalkohol, Stearylalkohol, Behenylalkohol, 2-Octyldodecanol und einer Mischung hiervon besteht, und ein Lösungsmittel umfasst und einen Wassergehalt von 20 % oder weniger aufweist, auf trockenes-Haar, das Erwärmen des Haares auf eine Temperatur von 38 bis 65°C für eine vorgeschriebene Dauer, wobei das Haar so bedeckt wird, dass keine Komponente der Haarbehandlungszusammensetzung entweichen kann, und das Auswaschen der Haarbehandlungszusammensetzung.

2. Verfahren zur Behandlung von Haar gemäss Anspruch 1, worin das Erwärmen des Haares für 3 Minuten oder länger durchgeführt wird.

3. Verfahren zur Behandlung von Haar gemäss Anspruch 1 oder 2, worin das Erwärmen des Haares mit einer Haarwärmehaube, die Heizbestandteile aufweist, durchgeführt wird.

## Revendications

1. Procédé non thérapeutique de traitement des cheveux qui comprend l'application, d'une composition pour le traitement des cheveux comprenant au moins un agent de type huile choisi dans le groupe constitué par les huiles de type ester, les huiles de type triglycéride, les huiles de type hydrocarbure, les huiles végétales, les huiles animales, le cétanol, l'alcool myristylique, l'alcool stéarylique, l'alcool béhénylique, le 2-octyldodécanol et un mélange de ceux-ci, et un solvant et ayant une teneur en eau de 20 % en poids ou plus basse aux cheveux secs, le chauffage des cheveux à une température allant de 38 à 65°C pendant une période de temps prescrite avec les cheveux qui sont couverts de manière à ne pas laisser s'échapper un constituant de la composition pour le traitement des cheveux, et l'enlèvement par lavage de la composition pour le traitement des cheveux.

2. Procédé de traitement des cheveux selon la revendication 1, dans lequel ledit chauffage des cheveux est effectué pendant 3 minutes ou plus longtemps.

3. Procédé de traitement des cheveux selon la revendication 1 ou 2, dans lequel le chauffage des cheveux est effectué avec un bonnet chauffant ayant des parties chauffantes.
